# FASCICULE DE BREVET EUROPEEN

(11) **EP 1 030 657 B1**
(45) Date de publication et mention de la délivrance du brevet: **10.10.2001**
(21) Numéro de dépôt: 98954553.8
(22) Date de dépôt: 10.11.1998
(51) Int. Cl.: A61K 9/70, A61K 7/48

(54) **ASSOCIATION PROLAMINES-LIPIDES POLAIRES VEGETAUX, SON PROCEDE DE PREPARATION ET SES APPLICATIONS**
KOMBINATION AUS PROLAMINEN UND POLAREN PFLANZLICHEN LIPIDEN, VERFAHREN ZU DEREN HERSTELLUNG SOWIE DEREN VERWENDUNGEN
PROLAMIN-PLANT POLAR LIPID COMBINATION, PREPARATION METHOD AND APPLICATIONS

(30) Priorité: 13.11.1997 FR 9714242
(43) Date de publication de la demande: 30.08.2000
(73) Titulaire: Laboratoires La Vipharm S.A., 75116 Paris (FR)
(72) Inventeur: FOTINOS, Spiros, GR-106 72 Athens (GR)
(74) Mandataire: Breese, Pierre
(86) Numéro de dépôt international: FR9802400
(87) Numéro de publication internationale: WO9925326

(56) Documents cités:
- EP-A- 0 704 200
- WO-A-91/15117
- WO-A-96/21462
- DE-A- 4 404 977
- FR-A- 2 710 649
- FR-A- 2 761 890

## Description

La présente invention concerne une association de protéines végétales et de lipides polaires végétaux, de préférence une association de protéines de céréales et de lipides polaires de céréales et de préférence encore une association de prolamines de blé et de lipides polaires de blé, destinée à être utilisée dans la délivrance de substances actives comprenant des agents thérapeutiques et des agents cosmétiques, à des êtres humains.

Des systèmes supports ou vecteurs pour différentes substances actives incluant des médicaments, des hormones, des peptides, des glycopeptides, des facteurs de croissance et similaires, sont bien connus dans la technique pour être faits de polymères synthétiques.

De tels systèmes particulièrement employés pour délivrer des agents actifs à la peau pour sa protection et son renouvellement, ou à une blessure pour la cicatriser et pour l'hémostase, ou encore sous forme d'un dispositif pour l'administration dermique, transdermique, mucosique et transmucosique, devraient satisfaire à des exigences telles que:
- être non toxiques,
- ne pas interrompre la délivrance d'une substance active au site d'application et
- ne pas interférer avec la vitesse de libération de la substance active,
- ne pas provoquer d'irritation,
- être enlevés facilement sans inconfort, lorsque cela est nécessaire,
- ne pas se détériorer, s'arracher ou se relâcher pendant la durée d'application, et
- de plus, ne pas laisser un résidu lorsqu'on les enlève, en particulier lorsqu'ils sont appliqués sur la peau.

Des polymères naturels, à savoir le collagène et la gélatine, ont également été utilisé comme agents d'encapsulation, vecteur, agents de support, dispensateurs, etc, pour délivrer des agents actifs.

Le collagène qui est un polymère biodégradable trouvé chez l'homme, a été utilisés pour la préparation d'un patch employé en chirurgie viscérale comme divulgué dans l'US 5,201,745, la préparation d'un pad ou bandage pour favoriser l'hémostase, traîter les tissus traumatisés ou accélérer la suture des tissus comme enseigné dans le WO-A-93/10731.

De façon similaire, des membranes de collagène ont été utilisées pour délivrer des substances actives comme divulgué dans l'EP-A-0 621 044, tandis que des pellicules ou films de collagène ont été proposés pour améliorer la délivrance contrôlée de médicaments comme décrit dans le WO-A-95/28964 et l'EP-A-0 518 697.

La chitine qui est un autre biopolymère abondant dans les crustacés, a été proposée pour obtenir l'hémostase comme divulgué dans l'US 4,394,373 et pour la préparation de bandages de la cornée et de lentilles de contact dans l'EP-A-0 486 294. La chitine et le chitosan qui est son produit de désacétylation, ont été proposés pour la préparation de formulations à libération contrôlée ou prolongée comme décrit dans l'EP-A-0 187 703.

En général, les polymères d'origine naturelle tels que ceux donnés ci-dessus, ne peuvent pas être considérés comme dépourvus de tout effet indésirable, en particulier ceux qui sont associés à l'induction d'une réponse immunitaire. Par exemple, l'antigénicité du collagène dépend principalement de l'espèce animale, de sorte qu'il y a toujours besoin de méthodes de traitement sophistiquées qui pourraient fournir un collagène moins antigénique. De façon similaire, les produits des coquilles marines sont aussi impliqués dans l'induction d'allergie chez les humains. De plus, une démonstration récente relate la transmission aux humains de maladies ayant surtout pour origine des espèces animales, après utilisation/consommation de produits animaux.

Par conséquent, il existe un besoin de disposer d'un polymère naturel pur et sûr dépourvu de toute propriété antigénique, qui puisse en outre être utilisé comme système vecteur pour des composés actifs.

La présente invention utilise un tel polymère naturel sous forme d'une nouvelle association de protéines et de lipides polaires, tous d'origine végétale, de préférence, une association de protéines de céréales et de lipides polaires végétaux et de préférence encore une association de prolamines et de lipides polaires de céréales.

De plus, l'invention concerne l'utilisation d'une telle association comme système vecteur sous forme d'une pellicule ou d'un patch pour l'application dermique (topique), transdermique, mucosique, transmucosique et aussi pour la cicatrisation et la protection de la peau, la cicatrisation et l'hémostase des blessures.

L'invention concerne également un système vecteur sous forme d'une pellicule ou d'un patch comprenant en outre des composés actifs et d'autres additifs qui optimisent les propriétés du système.

L'invention concerne principalement l'utilisation d'un polymère naturel, de préférence fait d'une protéine de type prolamine provenant du maïs ou du blé, c'est-à-dire la zéine ou la gliadine, respectivement. Dans la présente invention, la gliadine est préférée mais on n'envisage pas de limitation en ce qui concerne l'utilisation d'autres prolamines ou d'un mélange de celles-ci.

L'invention concerne également l'utilisation de lipides polaires d'origine végétale, de préférence l'utilisation de céramides végétaux ou de compositions de lipides polaires riches en céramides végétaux. Dans la présente invention les céramides végétaux et en particulier les céramides de céréales (glycosylés ou non, ou un mélange de ceux-ci) sont préférés mais on n'envisage pas de limitation en ce qui concerne l'utilisation d'autres lipides végétaux ou d'un mélange de ceux-ci.

L'invention concerne aussi l'utilisation d'une fraction de gliadines particulière, choisie de manière à optimiser les propriétés physicochimiques dudit polymère et en outre à améliorer son efficacité en tant que système vecteur.

L'invention concerne de préférence une nouvelle association gliadine-céramides végétaux utilisée comme système vecteur pour la délivrance de substances actives à des êtres humains par administration dermique, transdermique, mucosique et transmucosique et également comme système vecteur pour la protection et la cicatrisation de la peau, la cicatrisation et l'hémostase des blessures.

L'expression "substances actives" telle qu'utilisée ici comprend tout agent thérapeutique ou cosmétique.

De plus, l'expression "agent thérapeutique" telle qu'utilisée ici comprend tout composé non organique ou organique sans limitation, avec tant des propriétés hydrophiles qu'hydrophobes, connus dans la technique pour être utilisés pour le traitement de certains troubles.

De plus, l'expression "agent cosmétique" telle qu'utilisée ici, comprend tout composé connu dans la technique pour être utilisé pour améliorer l'apparence de la peau.

L'invention concerne encore un système vecteur sous forme d'une pellicule ou d'un patch adapté/ajusté sous des formes et des tailles multiples convenant pour son application sur un site choisi. L'expression "site choisi" telle qu'utilisée ici signifie toute partie de la peau ou de la muqueuse.

L'invention concerne encore une association prolamines-lipides polaires végétaux, plus particulièrement une association gliadine-céramides de céréales sous la forme d'une pellicule ou d'un patch utilisé comme système vecteur de médicaments, capable d'administrer les substances actives par voie systémique ou topique.

Les expressions "voie systémique" et "voie topique" telles qu'utilisées ici, signifient que les substances actives atteindront ou non la circulation systémique, respectivement.

L'invention concerne encore une association prolamines-lipides polaires de céréales, plus particulièrement une association gliadine-céramides de céréales, en particulier sous forme d'un patch, pour l'application directe sur la peau.

L'invention concerne encore une association prolamines-lipides polaires de céréales, plus particulièrement une association gliadine-céramides de céréales, en particulier sous forme d'un patch dans lequel ladite association est mise en sandwich entre une pellicule de renforcement et une couche amovible.

L'invention concerne encore une association prolamines-lipides polaires de céréales, plus particulièrement une association gliadine-céramides de céréales, en particulier sous la forme d'une pellicule, dont la formation est effectuée *in situ* sur la peau ou par un processus approprié.
L'invention concerne aussi une association prolamines-lipides polaires de céréales plus particulièrement une association gliadine-céramides de céréales, sous forme d'une pellicule ou d'un patch utilisé comme système vecteur de médicaments appliqué, sans limitation à ces applications, pour la délivrance transdermique de médicaments, ou la délivrance d'agents cosmétiquement actifs, ou la délivrance d'agents topiquement actifs tels que ceux pour traiter les maladies du derme et des ongles et les agents soulageant la douleur tels que ceux pour le traitement de l'arthrite, ou pour délivrer des agents à activité buccale tels que ceux qui sont appliqués avant et après les opérations dentaires, pour l'hygiène dentaire et pour le traitement systémique de différentes maladies à travers la muqueuse buccale ou la délivrance d'agents actifs à travers la muqueuse vaginale, nasale et oculaire.

L'invention concerne encore une association prolamines-lipides polaires de céréales, plus particulièrement une association gliadine-céramides de céréales sous forme d'une pellicule ou d'un patch utilisé comme système vecteur de médicaments appliqués, sans limitation, à la délivrance de médicaments pour la protection et la cicatrisation de la peau, la cicatrisation et l'hémostase des blessures.

L'invention concerne aussi une association prolamines-lipides polaires de céréales, plus particulièrement une association gliadine-céramides de céréales sous forme d'une pellicule ou d'un patch pour la délivrance topique ou transdermique de médicaments, qui est facilement produite et est stable pendant une longueur de temps souhaitée et sûre pour être portée par des êtres humains.

Le terme "stable" tel qu'utilisé ici signifie que, pendant la conservation, toutes les modifications chimiques qui pourraient avoir lieu n'affecteront pas son efficacité thérapeutique avant l'expiration d'une durée prédéterminée.

Le terme "sûre" tel qu'utilisé ici, signifie qu'aucun effet indésirable n'est induit lorsqu'elle est appliquée à des êtres humains.

Les figures 1 à 6 représentant différents types de patchs qui peuvent comprendre l'association prolamines-lipides polaires végétaux selon la présente invention, sont décrites plus loin, en relation avec une forme de réalisation de l'invention.

Les figures 7a et 7b montrent un ensemble de patchs circulaires dans un format qui convient à une utilisation prolongée par le patient.

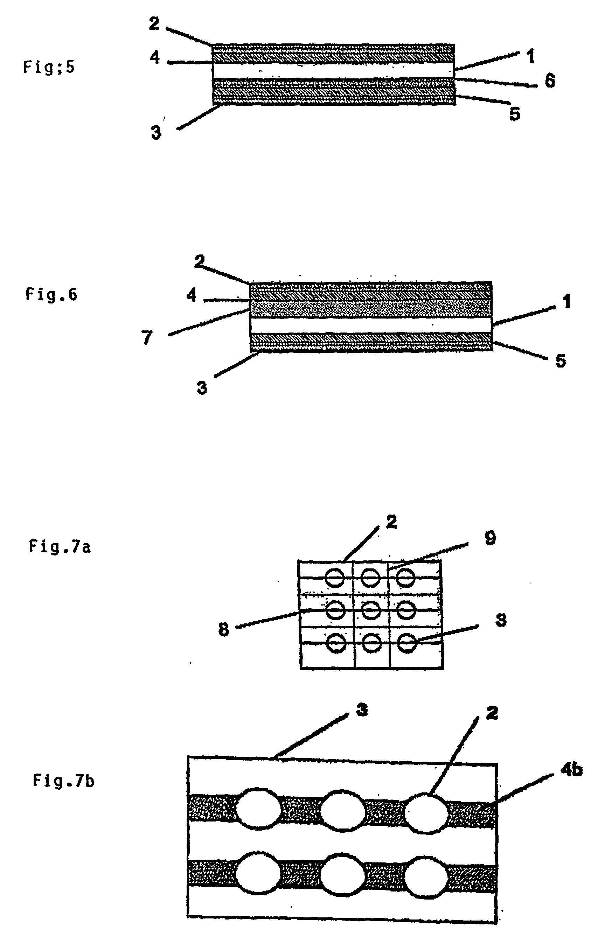

La figure 7a montre les lignes de perforation 9 gravées au milieu de l'espace des dispositifs, alors que les lignes de découpage 8 sont gravées horizontalement. Une plaquette rigide forme la doublure amovible, la dispersion de gliadine et de ceramides forme la matrice adhésive et la couche de renforcement est formée d'un matériau flexible. Un protocole de traitement pourrait comprendre l'utilisation en tandem de tous les patchs.

La figure 7b montre un ensemble de patchs circulaires dans lequel la doublure amovible est couchée par gravure au moyen d'une couche adhésive synthétique 4b de DuroTak® 72-8661.

Selon l'un de ses aspects, la présente invention fournit une association prolamines-lipides polaires végétaux, dans laquelle les composants prolamines et lipides polaires végétaux sont présents dans des proportions telles que des pelliculles stables puissent être obtenues avec cette association.

Les prolamines sont des protéines de réserve des céréales dont les polypeptides sont solubles dans des solutions hydro-alcooliques (n-propanol à 50-60% ou éthanol à 60-90%). Ces protéines se trouvent en abondance dans les céréales. Elles sont hydrophobes et se lient aux lipides et peuvent également interagir avec des molécules hydrophiles. Parmi les prolamines les plus courantes, se trouvent les gliadines du blé, les zéines du maïs, et les hordéines de l'avoine. Elles sont riches en glutamine et proline, et contiennent une faible proportion d'acides aminés basiques. Toutes ces protéines présentent un degré élevé d'homologie de séquence.

Des informations importantes existent dans la littérature en ce qui concerne les applications de telles protéines. Par exemple, des revêtements de prolamines pour les granulés, les comprimés, les agents nutritifs, etc, sont divulgués dans l'US-A-5,160,742, l'US-A-5,182,130, l'EP-A-0 090 559, le WO-A-96/21462 and le WO-A-93/12771. De plus, des pellicules de prolamines ont été utilisées dans des formulations de médicaments à libération prolongée ou contrôlée comme décrit dans l'US-A-4,137,300 et par Stella et al. dans Int. J. Pharmaceutics 121, 117-121, 1995.

Dans la présente invention, la gliadine qui provient du blé est préférée mais d'autres prolamines, sans limitation, ou des mélanges de celles-ci peuvent être utilisés.

Les lipides polaires de céréales tels qu'extraits des céréales comprennent des glycolipides, des phospholipides, des sphingolipides connus en tant que céramides et glycosylcéramides, et de faibles quantités de protéines et de lipides apolaires.

Selon un mode de réalisation préféré, l'invention fournit une formulation de gel hydro-alcoolique qui a la composition suivante :
- prolamines : de 20 à 40% p/p ;
- lipides polaires végétaux : de 0,1 à 5% p/p ;
- solution hydro-éthanolique (35 à 80°) : qsp 100%.

Selon un mode de réalisation plus préféré, les prolamines sont des prolamines de blé, à savoir des gliadines.

Le terme "gliadine" tel qu'utilisé dans la technique, représente un mélange de protéines de masse molaire relativement faible, classées en quatre groupes, en tant que α-, β-, γ- and ω-gliadines selon leur mobilité électrophorétique, c'est-à-dire en tant que protéines migrant rapidement à lentement. Les gliadines sont riches en acides aminés glutamine et proline mais contiennent des quantités moindres de lysine, méthionine et tryptophane. En particulier, la ω-gliadine contient la quantité la plus élevée de glutamine et de proline, tout en étant pauvre en acides aminés contenant du soufre tels que la méthionine et la cystéine.

Les gliadines en solution, et en particulier en solution hydro-alcoolique, présentent une configuration qui facilite les interactions avec tant les molécules hydrophobes que les molécules hydrophiles. De plus, les gliadines forment une solution visqueuse qui par traitement, peut être transformée en une pellicule. De plus, une pellicule sèche de gliadine retrouve ses propriétés élastomères par hydratation.

Les gliadines, sous forme d'une pellicule, ont déjà été proposées comme vecteurs ou supports pour la délivrance contrôlée ou prolongée de médicaments.

Selon un mode de réalisation particulièrement préféré, les prolamines de blé ou gliadines utilisées dans la présente invention, sous forme d'une poudre beige, ont la composition suivante (p/p)
- prolamines : 75 à 90% ;
- lipides : 1 à 4% ;
- sucres : 3 à 6% ;
- cendres contenant du soufre : 2 à 5% ;
- humidité : 5 à 10%.

Selon un autre mode préféré de réalisation, les lipides polaires végétaux contiennent une proportion élevée de céramides et de glycosylcéramides, ci-après "(glycosyl)céramides" et sont de préférence sous forme de produits extraits d'une fraction de céréales, de préférence une fraction de blé, ayant des concentrations élevées en (glycosyl)céramides et glycolipides, et enrichies en (glycosyl)céramides par purification (FR-A-91 06336; WO-A-92/21321).

La présence d'une forte proportion de (glycosyl)céramides est avantageuse du fait que les céramides (glycosylés ou non) sont des composants de la membrane cellulaire qui contribuent aux fonctions cellulaires. On les a trouvés dans la peau et dans d'autres organes vitaux dans le corps humain. De façon similaire, les céramides (glycosylés ou non) ont été isolés de plantes telles que le blé, les épinards, le riz, le soja et le millet.

Par exemple, la structure chimique d'un céramide glycosylé est constituée d'un acide gras, d'une longue base aliphatique telle que la sphingosine ou la phytosphingosine, et d'une partie sucre tel que le glucose. L'acide gras est lié à la base aliphatique par une liaison amide, tandis que la partie sucre est liée à la base aliphatique par une liaison glycosidique. Les céramides sont dépourvus de partie sucre.

Des études récentes sur l'évaluation des propriétés des (glycosyl)céramides et en particulier sur les céramides végétaux ont montré que ces composés
- ne sont pas cytotoxiques du fait qu'ils n'inhibent pas la prolifération cellulaire,
- sont des agents hautement humidifiants pour l'épiderme de la peau.

Ils présentent aussi
- un effet antiradicalaire,
- un effet anti-élastase du fait qu'ils protègent la génération de fibres élastiques et de collagène pour le tissu conjonctif comme décrit par Bizot-Foulon V. et al. dans Int. J. Cosmetic Science 17, 255-264, 1995.

Le potentiel des céramides et en particulier des céramides végétaux pour pénétrer l'épiderme a été examiné tant dans des études *in vivo* qu'*in vitro* en utilisant des échantillons de peau de cadavres et de la peau régénérée expérimentalement. Les résultats ont indiqué que l'on trouvait des céramides depuis l'épiderme jusqu'à la couche basale et en particulier dans l'espace intracellulaire entre les kératinocytes, alors qu'ils ne présentaient aucun passage systémique.

Une autre propriété des (glycosyl)céramides qui est d'un intérêt particulier dans la présente invention concerne leurs propriétés de vecteur ou support en augmentant la biodisponibilité des composés actifs pour l'épiderme.

Un produit purifié riche en (glycosyl)céramides, préparé à partir de blé et utilisé de préférence selon la présente invention, est sous la forme d'une poudre beige et a la composition suivante (p/p) :
- lipides polaires (glycolipides + phospholipides + céramides + glycosylcéramides) : 70 à 85% ;
- protéines : 4 à 7% ;
- lipides apolaires : 5 à 15% ;
- humidité : 1 à 3%.

Les extraits de céramides végétaux sont essentiellement composés de glycosylcéramides et de céramides tels que définis ci-dessus, la partie sphingosine étant une partie phytosphingosine.

Selon un autre mode de réalisation préféré de la présente invention, la formulation de gel hydro-alcoolique a la composition suivante :
- prolamines de blé : environ 31% p/p ;
- lipides polaires de blé : environ 1.3% p/p ;
- solution hydro-éthanolique (50°):qsp 100%.

Selon un autre de ses aspects, l'invention fournit un procédé pour la préparation de la formulation de gel hydro-alcoolique décrite ci-dessus, lequel procédé comprend les étapes consistant à :
- chauffer une solution hydro-éthanolique à une température de 42 à 48°C ;
- ajouter à cette solution la quantité correspondante de poudre de prolamines, en petites portions, sous agitation vigoureuse jusqu'à obtention d'une dispersion homogène ;
- ajouter à cette dispersion la quantité correspondante de lipides polaires végétaux, éventuellement avec les additifs ;
- agiter la dispersion à une température de 42 à 48°C pendant 20 à 40 minutes ; et
- laisser le mélange refroidir à la température ambiante sous agitation modérée.

L'association prolamines-lipides polaires végétaux (ci-après "association") sous la forme d'une pellicule ou d'un patch comme proposé dans la présente invention peut être produite sous des formes et des épaisseurs variées, selon le site de la peau/muqueuse sur lequel elle doit être appliquée et le type de traitement à utiliser.

Dans des modes préférés de réalisation de cette invention, l'association peut en outre comprendre des agents plastifiants qui peuvent améliorer les propriétés élastomères/viscoélastiques des prolamines, et des agents actifs connus dans la technique pour être utilisés pour l'administration dermique, transdermique, mucosique ou transmucosique et aussi pour la cicatrisation et la protection de la peau, la cicatrisation et l'hémostase des blessures.

Dans les autres modes de réalisation préférés de cette invention, l'association peut être produite sous forme d'une pellicule en utilisant un applicateur d'aérosols, ou une bille telle que celles employées dans l'application d'un déodorant, la pellicule étant formée sur la peau lorsque la solution hydroalcoolique s'évapore, ou en variante par préfabrication avant son application.

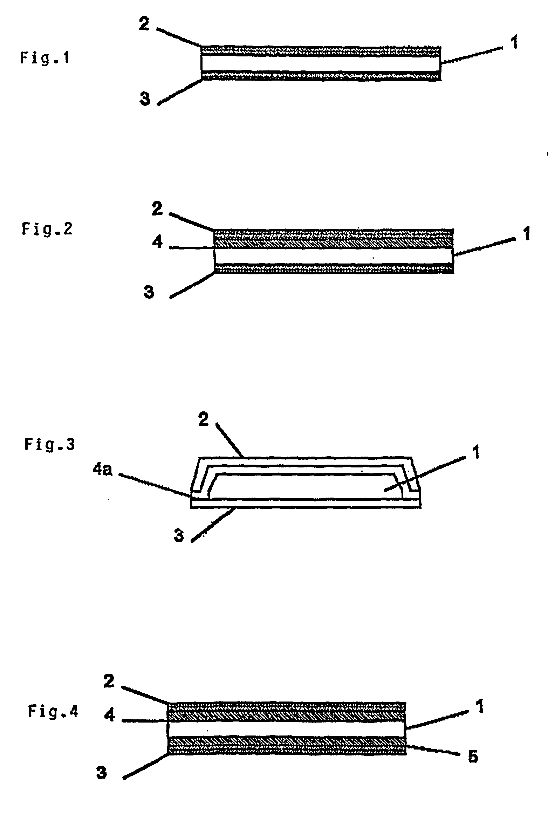

Dans un autre mode de réalisation de la présente invention, le patch contenant l'association tel que représenté à la figure 1, comprend l'association sous forme d'une couche 1 qui est insérée entre une pellicule de renforcement 2 et une couche amovible 3. La pellicule de renforcement 2 pourrait être stratifiée initialement avec une mince couche d'adhésif synthétique 4, comme représenté à la figure 2, ce qui facilite le dépôt d'une couche d'association dessus. De façon similaire, le coulage d'une couche d'association peut être effectué à travers un adhesif périphérique 4a comme montré à la figure 3. De plus, la couche amovible 3 pourrait aussi être stratifiée avec une mince couche d'adhésif synthétique 5 ou une membrane poreuse 6 comme présenté sur les figures 4 et 5, respectivement, l'adhésif synthétique 5 venant directement en contact avec la zone d'application choisie, après que la couche amovible 3 ait été arrachée.

Dans encore un autre mode de réalisation de la présente invention, l'association peut être mise sous forme d'un système multi-couche 1-7, contenant différentes concentrations d'agents actifs pour agir comme système de gradient pour optimiser l'efficacité du patch, comme illustré à la figure 6.

Dans d'autres modes de réalisation de la présente invention, les patchs montrés aux figures 1 et 2 peuvent être préparés sans couche amovible 3.

La couche de pellicule de renforcement peut être faite de matériau plastique, sous forme de tissu, tissé ou non tissé, poreux ou occlusif. De préférence, la pellicule de renforcement est toujours "apte à respirer", pour les applications comprenant la protection et la cicatrisation de la peau, la cicatrisation et l'hémostase des blessures.

La pellicule de renforcement peut être en tout matériau convenable connu dans la technique tel que du papier; de la cellophane ; des pellicules de plastique tel que du polyéthylène, polyester, polyuréthane, du poly(chlorure) de vinyle et du polyamide; des tissus et feuilles métalliques qui sont imperméables et ne réagissent pas avec les substances actives présentes dans la couche d'association. La pellicule de renforcement peut être composite, transparente, opaque ou couleur chair, ou aluminisée, ou avoir une combinaison de ces propriétés avec une épaisseur allant de 1 à 5 mils (1/1000 de pouce) (environ 25 à 130 µm) et peut être formée de tout matériau choisi parmi le CoTran™ 9720 (3M), le Saranex® (Dow Chemicals), une pellicule de polyester couleur chair Multilame 1009 (3M) ou tout autre matériau reconnu dans la technique comme ayant les propriétés souhaitables.

La couche amovible est placée contre la surface de la couche d'association, sur la surface opposée à la pellicule de renforcement. La couche amovible peut être faite de matériau imperméable à toute substance présente dans la couche d'association et est facilement enlevée avant l'utilisation. La couche facilement enlevable peut être faite de matériaux tels que du poly(chlorure) de vinyle, du polyester, du chlorure de polyvinylidène, du polystyrène, du polyéthylène, du papier etc. revêtu ou non d'un adhésif ou d'une membrane.

De préférence, la couche amovible est faite d'une pellicule de polystyrène à impact élevé, naturel (code de qualité : 10106) commercialisé par REXAM Release ou d'une pellicule de polyester siliconé commercialisé par REXAM Release. L'épaisseur de la couche facilement retirable est habituellement dans la gamme de 3 à 10 mils (environ 76 à 250 µm).

Les membranes utilisées pour revêtir la couche amovible stratifiée avec une mince couche d'adhésif synthétique peuvent être microporeuses ou semiperméables. Ces membranes peuvent être faites d'une pellicule de polyéthylène microporeuse ou d'une pellicule d'acétate d'éthylène-vinyle. De préférence, les membranes sont faites de CoTran® 9711 et CoTran® 9702 commercialisés par 3M.

Les agents thérapeutiques incorporés dans la couche d'association peuvent être différents agents hydrophiles et hydrophobes de toute catégorie therapeutique utilisée pour le traitement syst_mique ou topique, tels que des antimicrobiens comme la pénicilline, la tétracycline, l'érythomycine et similaires, des analgésiques tels que la morphine, la mépiridine et similaires, des antipyrétiques et des agents anti-inflammatoires tels que les salicylates, des dérivés d'indole et similaires, des antispasmodiques comme la scopolamine, l'atropine et similaires, des agents hormonaux tels que la cortisone, le cortisol, la calcitonine et similaires, des anesthésiques locaux tels que la procaïne, la lidocaïne, la xylocaïne et similaires, des médicaments sympathomimétiques tels que l'épinéphrine, l'amphétamine et similaires, des agents antiparasitaires tels que le métronidazole, le fenthion, le cythioate et similaires, des médicaments hypoglycémiques tels que l'insuline et les dérivés d'insuline et similaires, des agents anti-acné tels que l'acide salicylique, le peroxyde de benzoyle, l'acide rétinoïque et similaires, des agents nutritionnels tels que des vitamines, des acides aminés essentiels, des graisses essentielles et similaires, et d'autres agents actifs bénéfiques.

Les agents cosmétiques incorporés dans la couche d'association peuvent être tout agent choisi parmi les agents anti-hyperpigmentation, anti-taches, anti-vieillissement, pour le contour des yeux, ammincissants, anti-cellulite, anti-irritants adoucissants ou solaires, pour rafermir et étirer la peau, anti-élastase et anti-collagénase, des agents éliminant les radicaux libres, des séborégulateurs, des hydratants, et des produits spécifiques AHA (α-hydroxy acides) et. similaires, des vitamines et d'autres agents actifs bénéfiques.

Les agents actifs utilisés pour la protection et le renouvellement de la peau, la cicatrisation et l'hémostase des blessures peuvent être des anti-microbiens tels que la pénicilline, la tétracycline, l'érythromycine et similaires, des agents anti-inflammatoires tels que les salicylates, des dérivés d'indole et similaires, des agents hémostatiques tels que des phospolipides chargés négativement, des substances particulaires (kaolin) et similaires, des agents de croissance de la peau tels que des inhibiteurs de collagénase et similaires, des agents de cicatrisation des blessures tels que l'iodure de povidone, l'acide hyaluronique, et ses dérivés, l'huile de mastichinum provenant de Pistachia lentiscus var, Chia et similires, et tout autre composé connu dans la technique et considéré efficace pour le traitement des états mentionnés ci-dessus.

Lorsque cela est approprié, le système vecteur ou support de l'association n'est pas transformé en une pellicule mais la formation de la pellicule est effectuée directement sur le site de la peau. Par conséquent, le système vecteur ou support de l'association en solution contenant toutes les substances actives et d'autres additifs est pulvérisé sur la peau et la pellicule est formée après évaporation des solvants.

Avant l'application de la pellicule ou du patch d'association de l'invention sur un site de la peau, la peau doit être hydratée en utilisant de la solution saline normale ou de l'eau pour injection de sorte que le pouvoir d'adhésion de l'association soit restauré. Dans le cas des blessures, et en particulier des blessures exsudantes, il n'est pas nécessaire d'hydrater.

Dans la présente invention, le système vecteur ou support de l'association, sous forme d'une pellicule ou d'un patch, est finalement stérilisé en utilisant des techniques de radiation gamma comme décrit par les pharmacopées US et européennes.

Dans un autre mode de réalisation de la présente invention, la pellicule d'association peut être pliée et encapsulée dans une capsule dure pour la délivrance orale contrôlée de médicaments.

Les compositions de la présente invention peuvent aussi comprendre d'autres agents pharmaceutiquement acceptables tels que des solvants, des anti-oxydants, des plastifiants, des solubilisants, des agents favorisant la perméation à travers la peau, des humidifiants, des conservateurs, etc, connus dans la technique, de manière à améliorer les propriétés du système support ou vecteur introduit dans la présente invention.

Dans les modes de réalisation nouveaux et utiles qui viennent d'être décrits, le procédé pour préparer une pellicule ou un patch contenant l'association comprend le mélange des substances actives et des autres additifs avec l'association, celle-ci étant de préférence sous forme d'un gel. Dans le cas de la préparation d'un patch, dans un mode de réalisation préféré, le mélange est coulé sur une couche de polystyrène puis le système est stratifié sur une couche de polyester siliconé, revêtue au préalable d'une mince couche d'un adhésif acrylique.

L'invention n'est que simplement illustrée par les exemples qui suivent qui ne doivent pas être considérés comme limitant sa portée, étant donné que ces exemples et d'autres équivalents de ceux-ci apparaîtront aux hommes du métier à la lumière de la présente description et des dessins. La portée de l'invention est définie par les revendications annexées.

### EXEMPLE 1

### Préparation d'une poudre de prolamines de blé (gliadines), à partir de blé entier broyé.

Du blé broyé et de l'éthanol à 99°, en une proportion of 1:5 (p/p) sont introduits dans une cuve de 1500 litres et agités pendant 5 à 7 heures, en utilisant un agitateur à hélice. Le mélange est ensuite filtré soigneusement.

Le gâteau délipidé ainsi obtenu est extrait avec de l'éthanol à 80° dans une proportion de 1:6 (p/p) dans une cuve de 1500 litres en agitant jusqu'au lendemain avec un agitateur à hélice, à une température de 28 à 40°C. Le mélange est ensuite filtré en utilisant un filtre-presse.

Le nouveau gâteau ainsi obtenu est extrait de nouveau avec de l'éthanol à 80° dans une proportion de 1:5 (p/p) dans une cuve de 1500 litres en agitant jusqu'au lendemain avec un agitateur à hélice, à une température de 28 à 40°C. Le mélange est ensuite filtré en utilisant un filtre-presse.

Les filtrats sont combinés et concentrés sous vide jusqu'à évaporation complète de l'éthanol. De l'eau froide est ajoutée en une proportion de 1:3 (v/v) au concentrat contenant un mélange de prolamines de blé et d'eau. Après avoir laissé le mélange ci-dessus précipiter, on obtient une pâte humide.

Cette pâte humide est mélangée avec de l'acétone dans un rapport 1:5 (v/v), agitée vigoureusement (environ 3000 tr/min) pendant 20 à 40 minutes, puis filtrée sur Buchner.

Le gâteau de gliadines ainsi obtenu est rincé avec 3 volumes d'acétone, filtré sur Buchner sous vide, séché dans une étuve sous vide, broyé et micronisé.

Une fine poudre légèrement beige à beige est ainsi obtenue. Elle contient 75 à 90% de prolamines (méthode de Kjeldahl) et 1 à 2% de lipides. Sa composition en acides aminés est (% p/p) : arginine: 2,7; histidine: 2,3; lysine: 1,1; tyrosine: 3,2; tryptophane: 0,6; phénylalanine: 6,9; cystine: 2,6; thréonine: 2,1; sérine: 4,9; leucine: 6,5; méthionine: 1,7; acide aspartique: 1,3; isoleucine: 5,4; valine: 2,7; acide glutamique: 40,0; glycine: 0,5; alanine: 2,1; proline: 13,4.

### EXEMPLE 2

### Préparation d'un gel selon l'invention

30 à 35 g de la poudre de gliadines de l'exemple 1, appelée ci-après "gliadine", sont ajoutés en petites portions à 70 à 80 g d'une solution hydro-éthanolique (50°) chauffée à 42 à 48°C sous agitation vigoureuse jusqu'à obtention d'une dispersion homogène.

15 à 20 g des (glycosyl)céramides de blé purifiés décrits ci-dessus, appelés ci-après "céramides", sont ajoutés à cette dispersion. L'agitation et le chaufffage à 42 à 48°C sont poursuivis pendant 20 à 40 minutes. Puis on laisse le mélange refroidir à la température ambiante sous agitation modérée.

Finalement, on obtient un gel brun clair à brun foncé avec une viscosité de 800 à 1200 cp, et ayant la composition suivante (p/p) :
- teneur en gliadine : 27 à 30%;
- teneur en céramides : 1 à 2%.

### EXEMPLE 3

### Préparation d'un gel selon l'invention, qui contient du glycérol et du sorbitol comme plastifiants

### Exemple 3a :

Un gel "mère" d'association est préparé comme suit :
1. Preparer 604,63 g d'une solution alcooolique (EtOH:H₂O) à 50%.
2. Peser 272,08 g de poudre de gliadine et 12,09 g de poudre de céramides de céréales (pureté 50%).
3. Ajouter les poudres de gliadine et de céramides dans la solution alcoolique sous agitation vigoureuse à 42 à 48°C.
4. Peser séparément 33,25 g de glycérol (pureté 99%) et 77,94 g de sorbitol (pureté 70%) (proportion 3:7), mélanger dans un bécher pour obtenir une solution homogène.
5. Ajouter la solution précédente au mélange obtenu à l'étape 3.

### Exemple 3b :

Selon un autre mode de réalisation, un gel similaire peut être obtenu comme décrit à l'exemple 2 ci-dessus, si ce n'est que du glycérol et du sorbitol sont ajoutés ensemble avec la poudre de céramide dans des proportions telles que le gel ait la composition suivante (p/p) :
- teneur en prolamine: 27 à 30%;
- teneur en céramides: 1 à 2%;
- teneur en glycérol: 2.5 à 5%;
- teneur en sorbitol: 6 à 9%.

### EXEMPLE 4

### Préparation d'un patch d'association gliadine-céramides

La préparation d'un patch comprenant une association gliadine-céramides est décrite ci-dessous :

### 1. Préparation du mélange à base de l'association gliadine-céramides

Une composition d'un tel mélange utilisé pour la préparation d'un dispositif pour l'application topique directement sur la peau est donnée dans le Tableau 1.

**TABLEAU 1.**

| Composition du mélange | |
|---|---|
| COMPOSANT | QUANTITE % p/p |
| Gel de l'Exemple 3 | 89,08 |
| Agent anti-acné | 10,00 |
| Phenonip® * | 0,45 |
| Sorbate de potassium | 0,05 |
| D,L-α-Tocophérol | 0,42 |

| | |
|---|---|
| * Phenonip est un mélange de phénoxyéthanol, méthylparaben, éthylparaben, propylparaben et butylparaben. | |

La préparation du mélange à base de gliadine est comme suit:

Le sorbate potassium (0,05 g) est ajouté à un mélange d'agent anti-acné (10 g), de Phenonip (0,45 g) et de D,L-α-tocophérol (0,42 g dissous dans de l'éthanol à 50%) et le tout est agité dans les conditions ambiantes jusqu'à ce que tous les ingrédients soient complètement dissous. Ce mélange est encore dissous dans une partie d'une portion de 89,08 g de gel (contenant, sur une base sèche, 73% de poudre de gliadine, 3% de poudre de céramides, 15% de sorbitol et 9% de glycérol) et agité pendant 15 min dans les conditions ambiantes, jusqu'à obtention d'un mélange homogène. Ensuite, le reste du gel est ajouté et le mélange est agité encore pendant 15 min. Le système est laissé de côté pendant une courte durée pour que les bulles d'air soient complètement éliminées.

### 2. Coulage d'un adhésif synthétique sur la pellicule de renforcement

En utilisant un dispositif de revêtement approprié (outil carré Multi Clearance Applicator, commercialisé par BYK gardner) avec un écartement de coulée de 5 mils (environ 130 µm), une couche d'un adhésif synthétique tel que du DuroTak 87-2353 est déposée en revêtement sur une pellicule de polyester siliconé et séchée dans une étuve à 70-75°C pendant 15 à 18 minutes. Une pellicule de polyéthylène basse densité est ensuite stratifiée sur la pellicule d'adhésif synthétique. Le système est mis de côté.

### 3. Préparation d'un revêtement d'association

En utilisant un dispositif de revêtement approprié (outil carré Multi Clearance Applicator, commercialisé par BYK gardner) avec un écartement de coulée de 5 mils (environ 130 µm), une couche du mélange obtenu à la section 1 est déposée en revêtement sur le site non siliconé d'une pellicule de polystirène et séchée dans une étuve à 60-62°C pendant 10 à 12 minutes.

Le stratifié contenant le mélange est ensuite stratifié avec le système décrit à la section 2, après avoir retiré la pellicule de polyéthylène.

Le stratifié multi-couche ainsi obtenu est découpé pour former un dispositif ayant toute taille et forme souhaitable.

Dans un mode de réalisation de la présente invention, on fait passer la couche amovible à travers le Flexomaster 1B (commercialisé par ALLIED GEAR) et on découpe des patchs circulaires de 0,5" (environ 1,3 cm) de diamètre en laissant la couche amovible non pénétrée. Des lignes de découpe 8 et de perforations 9 sont simultanément gravées selon le mode perpendiculaire. Comme montré à la figure 7a, les lignes de perforation sont gravées au milieu de l'écartement des dispositifs, tandis que les lignes de découpe sont gravées horizontalement sur les couches amovibles.

### EXAMPLE 5

### Préparation d'un patch d'association gliadine-céramides contenant un agent de blanchiment de la peau.

Le mélange utilisé dans cet exemple a la composition suivante :

**TABLE 2.**

| Composition du mélange | |
|---|---|
| COMPOSANT | QUANTITE % p/p |
| Gel de l'Exemple 3 | 87,00 |
| Etioline¹ | 5,00 |
| Gatuline Whitening² | 5,00 |
| Palmitate d'ascorbyle | 2,00 |
| D,L-α-Tocophérol | 1,00 |

| | |
|---|---|
| 1. L' Etioline est un extrait de plante africaine (Matricarpe du genre Spermacocea), qui peut inhiber la tyrosinase, une enzyme responsable de la synthèse de la mélanine. | |
| 2. La Gatuline whitening obtenue par fermentation des acides kojique et lactique, est un inhibiteur de la tyrosinase. | |

Le reste du processus est le même que dans l'exemple 4.

### EXEMPLE 6

### Préparation d'un patch d'association gliadine-céramides contenant de l'anesthésique local

Le mélange utilisé dans cet exemple a la composition suivante :

**TABLEAU 3.**

| Composition du mélange | |
|---|---|
| COMPOSANT | QUANTITE % p/p |
| Gel de l'exemple 3 | 98,80 |
| Lidocaïne HCl | 1,00 |
| Digluconate de chlorhexidine | 0,20 |

Le reste du processus est le même que dans l'exemple 4.

### EXEMPLE 7

### Préparation d'un patch d'association gliadine-céramides contenant des agents de raffermissement/tension (lifting) de la peau

Le mélange utilisé dans cet exemple a la composition suivante' :

**TABLEAU 4.**

| Composition du mélange | |
|---|---|
| COMPOSANT | QUANTITE % p/p |
| Gel de l'exemple 3 | 89,09 |
| Gatuline lifting ¹ | 3,00 |
| Palmitate de rétinyle | 2,00 |
| Gatuline RC ² | 5,00 |
| D,L-α-Tocophérol | 0,17 |
| Transcutol | 0,74 |

| | |
|---|---|
| 1. Gatuline lifting est un extrait végétal contenant des flavonoïdes, des tannins et une fraction protéique ayant des propriétés similaires de celles de la sérum albumine bovine. | |
| 2. Gatuline RC est un extrait de bourgeons de hêtre contenant des flavonoïdes, des peptides tels que des phytostimulines et autres. | |

Le reste du processus est le même que dans l'exemple 4.

### EXEMPLE 8

### Préparation d'un patch d'association gliadine-céramides contenant un agent kératolytique

Le mélange utilisé dans cet exemple a la composition suivante :

**TABLEAU 5.**

| Composition du mélange | |
|---|---|
| COMPOSANT | QUANTITE % p/p |
| Gel de l'exemple 3 | 99,50 |
| Acide salicylique | 0,50 |

Le reste du processus est le même que dans l'exemple 4.

### EXEMPLE 9

### Préparation d'un patch d'association gliadine-céramides pour le renouvellement de la peau

Une composition du mélange utilisé pour la préparation d'un patch pour l'application topique sur la peau est donnée dans le Tableau 6.

**TABLE 6.**

| Composition du mélange | |
|---|---|
| COMPOSANT | QUANTITE % p/p |
| Gel de l'exemple 3 | 98,58 |
| Inhibiteur de la collagénase | 0,20 |
| Phenonip® * | 0,75 |
| Sorbate de potassium | 0,05 |
| D,L-α-Tocophérol | 0,42 |

| | |
|---|---|
| * Phenonip est un mélange de phénoxyéthanol, méthylparaben, éthylparaben, propylparaben et butylparaben | |

Le reste du processus est le même que dans l'exemple 4.

### EXEMPLE 10

### Préparation d'un patch d'association gliadine-céramides pour la cicatrisation des blessures

Une composition du mélange utilisé pour la préparation d'un patch pour l'application topique sur la peau est donnée dans le Tableau 7.

**TABLEAU 7.**

| Composition du mélange | |
|---|---|
| COMPOSANT | QUANTITE % p/p |
| Gel de l'exemple 3 | 92,58 |
| Huile de Mastichinum | 5,00 |
| α-Bisabolol | 1,20 |
| Phenonip® | 0,75 |
| Sorbate de potassium | 0,05 |
| D,L α-Tocophérol | 0,42 |

Le reste du processus est le même que dans l'exemple 4.

Une autre composition pour la cicatrisation des blessures est présentée dans le tableau suivant :

**TABLEAU 8.**

| Composition du mélange | |
|---|---|
| COMPOSANT | QUANTITE % p/p |
| Gel de l'exemple 3 | 95,00 |
| Iodure de povidone | 5,00 |

### EXEMPLE 11

### Préparation d'un patch d'association gliadine-céramides contenant un agent de blanchiment de la peau.

Le mélange à base de gliadine utilisé dans cet exemple a la composition suivante :

**TABLEAU 9.**

| Composition du mélange | |
|---|---|
| COMPOSANT | QUANTITE % p/p (sur une base sèche) |
| Poudre de gliadine | 59,87 |
| Poudre de céramides | 2,66 |
| Sorbitol | 17,14 |
| Glycérol | 7,33 |
| Etioline | 5,00 |
| Gatuline Whitening | 5,00 |
| Palmitate d'ascorbyle | 2,00 |
| D,L-α-Tocophérol | 1,00 |

Le reste du processus est le même que dans l'exemple 4.

### EXEMPLE 12

### Préparation d'un patch d'association gliadine-céramides contenant de l'anesthésique local

Le mélange à base de gliadine utilisé dans cet exemple a la composition suivante :

**TABLEAU 10.**

| Composition du mélange | |
|---|---|
| COMPOSANT | QUANTITE % p/p (sur une base sèche) |
| poudre de gliadine | 68,00 |
| poudre de céramide | 3,00 |
| Sorbitol | 19,50 |
| Glycérol | 8,30 |
| Lidocaïne HCl | 1,00 |
| Digluconate de chlorhéxidine | 0,20 |

Le reste du processus est le même que dans l'exemple 4.

### EXEMPLE 13

### Préparation d'un patch d'association gliadine-céramides contenant des agents de rafermissement/tension (lifting) de la peau

Le mélange à base de gliadine utilisé dans cet exemple a la composition suivante :

**TABLEAU 11.**

| Composition du mélange | |
|---|---|
| COMPOSANT | QUANTITE % p/p (sur une base sèche) |
| poudre de gliadine | 61,31 |
| poudre de céramides | 2,73 |
| Sorbitol | 17,55 |
| Glycérol | 7,50 |
| Gatuline lifting | 3,00 |
| Palmitate de rétinyle | 2,00 |
| Gatuline RC | 5,00 |
| D,L-α-Tocophérol | 0,17 |
| Transcutol | 0,74 |

Le reste du processus est le même que dans l'exemple 4.

### EXEMPLE 14

### Préparation d'un patch d'association gliadine-céramides contenant un agent kératolytique

Le mélange à base de gliadine utilisé dans cet exemple a la composition suivante :

**TABLEAU 12.**

| Composition du mélange | |
|---|---|
| COMPOSANT | QUANTITE % p/p (sur une base sèche) |
| Poudre de gliadine | 68,48 |
| Poudre de céramides | 3,04 |
| Sorbitol | 19,60 |
| Glycérol | 8,38 |
| Acide salicylique | 0,50 |

Le reste du processus est le même que dans l'exemple 4.

### EXEMPLE 15

### Préparation d'un patch d'association gliadine-céramides pour le renouvellement de la peau

Une composition du mélange utilisé pour la préparation d'un patch pour l'application topique sur la peau est donnée dans le Tableau 13.

**TABLEAU 13.**

| Composition du mélange | |
|---|---|
| COMPOSANT | QUANTITE % p/p (sur une base sèche) |
| Poudre de gliadine | 67,84 |
| Poudre de céramide | 3,02 |
| Sorbitol | 19,42 |
| Glycérol | 8,30 |
| Inhibiteur de collagénase | 0,20 |
| Phenonip® | 0,75 |
| Sorbate de potassium | 0,05 |
| D,L-α-Tocophérol | 0,42 |

Le reste du processus est le même que dans l'exemple 4.

### EXAMPLE 16

### Préparation d'un patch d'association gliadine-céramides cour la cicatrisation des blessures

Une composition du mélange utilisé pour la préparation d'un patch pour l'application topique sur la peau est donnée dans le Tableau 14.

**TABLEAU 14.**

| Composition du mélange | |
|---|---|
| COMPOSANT | QUANTITE % p/p (sur une base sèche) |
| Poudre de gliadine | 63,71 |
| Poudre de céramide | 2,83 |
| Sorbitol | 18,24 |
| Glycérol | 7,80 |
| Huile de Mastichinum | 5,00 |
| α-Bisabolol | 1,20 |
| Phenonip® | 0,75 |
| Sorbate de potassium | 0,05 |
| D,L α-Tocophérol | 0,42 |

Le reste du processus est le même que dans l'exemple 4.

Une autre composition pour la cicatrisation des blessures est présentée dans le tableau suivant :

**TABLE 15.**

| Composition du mélange | |
|---|---|
| COMPOSANT | QUANTITE % p/p (sur une base sèche) |
| poudre de gliadine | 65,38 |
| Poudre de céramide | 2,91 |
| Sorbitol | 18,71 |
| Glycérol | 8,00 |
| Iodure de povidone | 5,00 |

## Revendications

1. Association prolamines-lipides polaires végétaux dans laquelle les composants prolamines et lipides polaires végétaux sont présents dans des proportions telles que des pellicules stables puissent être obtenues avec cette association.

2. Association prolamines-lipides polaires végétaux selon la revendication 1, dans laquelle l'association est sous la forme d'une formulation de gel hydro-alcoolique comprenant:
- prolamines: de 20 à 40% p/p ;
- lipides polaires végétaux : de 0,1 à 5% p/p ;
- solution hydro-éthanolique (35 à 80°): qsp 100%.

3. Association prolamines-lipides polaires végétaux selon la revendication 2, dans laquelle la formulation de gel hydro-alcoolique consiste en :
- prolamines de blé :environ 31% p/p ;
- lipides polaires de blé : environ 1,3% p/p ;
- solution hydro-éthanolique (50°) : qsp 100%.

4. Association prolamines-lipides polaires végétaux selon l'une quelconque des revendications 1 à 3, qui comprend en outre des agents plastifiants.

5. Association prolamine-lipides polaires végétaux selon la revendication 4, dans laquelle l'association est sous forme d'un gel comprenant (p/p):
- prolamines : 27 à 30% ;
- céramides : 1 à 2% ;
- glycérol: 2,5 à 5% ;
- sorbitol: 6 à 9%.

6. Association prolamines-lipides polaires végétaux selon la revendication 4 ou 5, dans laquelle la formulation de gel hydro-alcoolique comprend en outre des substances actives.

7. Association prolamines-lipides polaires végétaux selon la revendication 6, dans laquelle les substances actives comprennent des agents thérapeutiques et des agents cosmétiques.

8. Association prolamines-lipides polaires végétaux selon l'une quelconque des revendications 1 à 7, dans laquelle la prolamine est une prolamine de blé, à savoir la gliadine.

9. Association prolamines-lipides polaires végétaux selon la revendication 8, dans laquelle la gliadine, sous forme de poudre, a la composition suivante (p/p) :
- prolamines: 75 à 90% ;
- lipides: 1 à 4% ;
- sucres: 3 à 6% ;
- cendres à base de soufre: 2 à 5% ;
- humidité : 5 à 10%.

10. Association prolamines-lipides polaires végétaux selon l'une quelconque des revendications 1 à 9, dans laquelle les lipides polaires végétaux contiennent une proportion élevée de céramides et de glycosylcéramides.

11. Association prolamines-lipides polaires végétaux selon la revendication 10, dans laquelle les lipides polaires végétaux sont des lipides polaires de céréales, de préférence des lipides polaires de blé.

12. Association prolamines-lipides polaires végétaux selon la revendication 11, dans laquelle les lipides polaires de blé ont la composition suivante (p/p) :
- lipides polaires (glycolipides + phospholipides + céramides + glycosylcéramides): 70 à 85% ;
- protéines: 4 à 7% ;
- lipides apolaires: 5 à 15% ;
- humidité : 1 à 3%.

13. Procédé pour la préparation de la formulation de gel hydro-alcoolique selon l'une quelconque des revendications 2 à 12, comprenant :
- le chauffage d'une solution hydro-éthanolique à une température de 42 à 48°C ;
- l'addition à cette solution de la quantité correspondante de poudre de prolamines en petites portions, sous agitation vigoureuse jusqu'à obtention d'une dispersion homogène;
- l'addition à cette dispersion de la quantité correspondante de poudre de lipides polaires végétaux, éventuellement avec les additifs ;
- l'agitation de la dispersion à une température de 42 à 48°C pendant 20 à 40 minutes ; et
- l'abandon du mélange pour refroidir à la température ambiante, sous agitation modérée.

14. Pellicule comprenant l'association prolamines-lipides polaires végétaux selon l'une quelconque des revendications 1 à 12.

15. Patch comprenant l'association prolamines-lipides polaires végétaux selon l'une quelconque des revendications 1 à 12.

16. Aérosol comprenant l'association prolamines-lipides polaires végétaux selon l'une quelconque des revendications 1 à 12.

17. Application de la pellicule selon la revendication 14, dans le domaine des produits pharmaceutiques et des produits cosmétiques

18. Application de la pellicule selon la revendication 14 en tant que support ou vecteur pour des substances actives dans le domaine des produits pharmaceutiques et des produits cosmétiques.

19. Application du patch selon la revendication 15, dans le domaine des produits pharmaceutiques et des produits cosmétiques.

20. Application du patch selon la revendication 15, en tant que support ou vecteur pour des substances actives dans le domaine des produits pharmaceutiques et des produits cosmétiques.

21. Application de l'aérosol selon la revendication 16, dans le domaine des produits pharmaceutiques et des produits cosmétiques.

22. Application de l'aérosol selon la revendication 16, en tant que support ou vecteur pour des substances actives dans le domaine des produits pharmaceutiques et des produits cosmétiques.

## Patentansprüche

1. Vereinigung von Prolaminen und polaren pflanzlichen Lipiden, in denen die Bestandteile Prolamine und polare pflanzliche Lipide in solchen Verhältnissen vorhanden sind, daß mit dieser Vereinigung stabile Filme erhalten werden können.

2. Vereinigung von Prolaminen und polaren pflanzlichen Lipiden nach Anspruch 1, in denen die Vereinigung in der Form einer Formulierung eines hydroalkoholischen Gels ist, das folgendes enthält:
- Prolamine: 20 bis 40 Gewichtsprozente;
- polare pflanzliche Lipide: 0,1 bis 5 Gewichtsprozent;
- wäßrige Ethanollösung (35 bis 80°): ad 100%.

3. Vereinigung von Prolaminen und polaren pflanzlichen Lipiden nach Anspruch 2, in der die Formulierung des hydroalkoholischen Gels aus folgendem besteht:
- Weizenprolamine: ungefähr 31 Gewichtsprozente;
- polare Lipide des Weizens: ungefähr 1,3 Gewichtsprozent;
- wässrige Ethanollösung (50°): ad 100%.

4. Vereinigung von Prolaminen und polaren pflanzlichen Lipiden nach irgendeinem der Ansprüche 1 bis 3, die zusätzlich noch Weichmacher enthält.

5. Vereinigung von Prolaminen und polaren pflanzlichen Lipiden nach Anspruch 4, in der die Vereinigung in der Form eines Gels ist, das folgendes enthält (Angaben in Gewichtsprozenten):
- Prolamine: 27 bis 30%;
- Ceramide: 1 bis 2%;
- Glycerin: 2,5 bis 5%;
- Sorbitol: 6 bis 9%.

6. Vereinigung von Prolaminen und polaren pflanzlichen Lipiden nach Anspruch 4 oder 5 in der die Formulierung des hydroalkoholischen Gels zusätzlich noch aktive Substanzen enthält.

7. Vereinigung von Prolaminen und polaren pflanzlichen Lipiden nach Anspruch 6, in der die aktiven Substanzen therapeutische und kosmetische Wirkstoffe beinhalten.

8. Vereinigung von Prolaminen und polaren pflanzlichen Lipiden nach irgendeinem der Ansprüche 1 bis 7, in der das Prolamin ein Weizenprolamin ist, genauer gesagt Gliadin.

9. Vereinigung von Prolaminen und polaren pflanzlichen Lipiden nach Anspruch 8 in der das Gliadin pulverförmig ist und die folgende Zusammensetzung hat(Gewichtsprozente):
- Prolamine: 75 bis 90%;
- Lipide: 1 bis 4%;
- Zucker: 3 bis 6%;
- Asche auf Schwefelbasis: 2 bis 5%;
- Feuchtigkeit: 5 bis 10%.

10. Vereinigung von Prolaminen und polaren pflanzlichen Lipiden nach irgendeinem der Ansprüche 1 bis 9, in der die polaren pflanzlichen Lipide einen hohen Anteil Ceramide und Glycosylceramide enthalten.

11. Vereinigung von Prolaminen und polaren pflanzlichen Lipiden nach Anspruch 10, in der die polaren pflanzlichen Lipide polare Getreidelipide, vorzugsweise polare Weizenlipide sind.

12. Vereinigung von Prolaminen und polaren pflanzlichen Lipiden nach Anspruch 11, in welcher die polaren Weizenlipide die folgende Zusammensetzung haben (Gewichtsprozente):
- polare Lipide (Glykolipide + Phospholipide + Ceramide + Glykosylceramide: 70 bis 85%;
- Proteine: 4 bis 7%;
- unpolare Lipide: 5 bis 15%;
- Feuchtigkeit: 1 bis 3%.

13. Verfahren zur Herstellung der Formulierung von hydroalkoholischem Gel nach irgendeinem der Ansprüche 2 bis 12, das beinhaltet:
- das Erhitzen einer wäßrigen Ethanollösung auf eine Temperatur von 42 bis 48°C;
- das Zugeben der entsprechenden Menge Prolaminpulver in kleinen Portionen unter kräftigem Rühren bis zum Erreichen einer homogenen Dispersion
- das Zugeben der entsprechenden Menge Pulver von polaren pflanzlichen Lipiden, evtl. mit Zusatzstoffen, zu dieser Dispersion;
- Das Rühren der Dispersion bei einer Temperatur von 42 bis 48°C während 20 bis 40 Minuten; und
- das Stehenlassen der Mischung zum Abkühlen auf Umgebungstemperatur, unter leichtem Rühren.

14. Film, der eine Vereinigung von Prolaminen und polaren pflanzlichen Lipiden nach irgendeinem der Ansprüche 1 bis 12 beinhaltet.

15. Patch, der eine Vereinigung von Prolaminen und polaren pflanzlichen Lipiden nach irgendeinem der Ansprüche 1 bis 12 beinhaltet.

16. Aerosol, das eine Vereinigung von Prolaminen und polaren pflanzlichen Lipiden nach irgendeinem der Ansprüche 1 bis 12 beinhaltet.

17. Anwendung des Films nach Anspruch 14 im Bereich der pharmazeutischen und kosmetischen Produkte.

18. Anwendung des Films nach Anspruch 14 als Trägermaterial oder Vehikel für aktive Substanzen im Bereich der pharmazeutischen und kosmetischen Produkte.

19. Anwendung des Patchs nach Anspruch 15 im Bereich der pharmazeutischen und kosmetischen Produkte.

20. Anwendung des Patchs nach Anspruch 15 als Trägermaterial oder Vehikel für aktive Substanzen im Bereich der pharmazeutischen und kosmetischen Produkte.

21. Anwendung des Aerosols nach Anspruch 16 im Bereich der pharmazeutischen und kosmetischen Produkte.

22. Anwendung des Aerosols nach Anspruch 16 als Trägermaterial oder Vehikel für aktive Substanzen im Bereich der pharmazeutischen und kosmetischen Produkte.

## Claims

1. Prolamin-plant polar lipid association wherein the prolamin and plant polar lipid constituents are present in proportions such that stable particles can be obtained with said association.

2. Prolamin-plant polar lipid association according to claim 1, wherein the association is in the form of a hydroalcoholic gel formulation comprising:
- prolamins: 20 to 40% w/w;
- plant polar lipids: 0.1 to 5% w/w;
- hydro-ethanol solution (35 to 80°): up to 100%.

3. Prolamin-plant polar lipid association according to claim 2, wherein the hydroalcoholic gel formulation consists of:
- wheat prolamins: approximately 31% w/w;
- wheat polar lipids: approximately 1.3% w/w;
- hydro-ethanol solution (50°): up to 100%.

4. Prolamin-plant polar lipid association according to any of claims 1 to 3, which also comprises plasticising agents.

5. Prolamin-plant polar lipid association according to claim 4, wherein the association is in the form of gel comprising (w/w):
- prolamins: 27 to 30%;
- ceramides: 1 to 2%;
- glycerol: 2.5 to 5%;
- sorbitol: 6 to 9.

6. Prolamin-plant polar lipid association according to claim 4 or 5, wherein the hydroalcoholic gel formulation also comprises active substances.

7. Prolamin-plant polar lipid association according to claim 6, wherein the active substances comprise therapeutic agents and cosmetic agents.

8. Prolamin-plant polar lipid association according to any of claims 1 to 7, wherein the prolamin is a wheat prolamin, namely gliadin.

9. Prolamin-plant polar lipid association according to claim 8, wherein the composition of gliadin, in powder form, is as follows (w/w):
- prolamins: 75 to 90%;
- lipids: 1 to 4%;
- sugars: 3 to 6%;
- sulphur-based ash: 2 to 5%;
- moisture: 5 to 10%.

10. Prolamin-plant polar lipid association according to any of claims 1 to 9, wherein the plant polar lipids contain a high proportion of ceramides and glycosylceramides.

11. Prolamin-plant polar lipid association according to claim 10, wherein the plant polar lipids are cereal polar lipids, preferentially wheat polar lipids.

12. Prolamin-plant polar lipid association according to claim 11, wherein the composition of the wheat polar lipids is as follows (w/w):
- polar lipids (glycolipids + phospholipids + ceramides + glycosylceramides): 70 to 85%;
- proteins: 4 to 7%;
- apolar lipids: 5 to 15%;
- moisture: 1 to 3%.

13. Method to prepare the hydroalcoholic gel formulation according to any of claims 2 to 12, comprising:
- heating of a hydro-ethanol solution to a temperature of 42 to 48°C;
- addition of said solution to the corresponding quantity of prolamin powder in small portions, with vigorous stirring until a homogeneous dispersion is obtained;
- addition to said dispersion of the corresponding quantity of plant polar lipid powder, with additives if required;
- stirring of the dispersion at a temperature of 42 to 48°C for 20 to 40 minutes; and
- leaving the mixture to cool at ambient temperature, with moderate stirring.

14. Film comprising the prolamin-plant polar lipid association according to any of claims 1 to 12.

15. Patch comprising the prolamin-plant polar lipid association according to any of claims 1 to 12.

16. Aerosol comprising the prolamin-plant polar lipid association according to any of claims 1 to 12.

17. Application of the film according to claim 14, in the field of pharmaceutical products and cosmetic products.

18. Application of the film according to claim 14 as a substrate or vector for active substances in the field of pharmaceutical products and cosmetic products.

19. Application of the patch according to claim 15, in the field of pharmaceutical products and cosmetic products.

20. Application of the patch according to claim 15 as a substrate or vector for active substances in the field of pharmaceutical products and cosmetic products.

21. Application of the aerosol according to claim 16, in the field of pharmaceutical products and cosmetic products.

22. Application of the aerosol according to claim 16 as a substrate or vector for active substances in the field of pharmaceutical products and cosmetic products.
